# EUROPÄISCHE PATENTSCHRIFT

(11) **EP 0 289 905 B1**
(45) Veröffentlichungstag und Bekanntmachung des Hinweises auf die Patenterteilung: **10.03.1993**
(21) Anmeldenummer: 88106611.2
(22) Anmeldetag: 25.04.1988
(51) Int. Cl.: A61N 1/04, A61B 5/0408

(54) **Saugelektrode**
Suction electrode
Electrode fixée par aspiration

(30) Priorität: 08.05.1987 DE 8706664 U; 08.05.1987 DE 8706665 U
(43) Veröffentlichungstag der Anmeldung: 09.11.1988
(73) Patentinhaber: SIEMENS AKTIENGESELLSCHAFT, 80333 München (DE)
(72) Erfinder: Sieber, Gotthold, Dipl.-Ing.(FH), D-8520 Erlangen (DE)

(56) Entgegenhaltungen:
- EP-A- 0 199 694
- CH-A- 301 242
- DE-A- 2 548 805
- DE-B- 1 939 523
- DE-B- 2 925 909
- DE-C- 3 300 765
- DE-U- 7 935 086
- ELEKTRO MEDICA, Band 39, Nr. 4, April 1971, Erlangen, DE; G. HENKE: "Vereinfachte und sichere Elektrodenanlage in der Reizstrombehandlung durch Appliserv"

## Beschreibung

Die Erfindung bezieht sich auf eine Saugelektrode mit einem Elektrodengehäuse, einer elektrisch leitenden Applikationselektrode, einer Unterdruckdüse und einer Saugglocke aus elastischem Material, wobei die Unterdruckdüse einen Kanal aufweist, der zumindest an einer Stelle enger als an anderen Stellen zur Erzeugung eines Unterdrucks beim Durchführen eines Mediums ausgebildet und mit einem in Höhe der engen Stelle mündenden Ansaugkanal versehen ist.

Durch die DE-B-19 39 523 ist eine Saugelektrode dieser Art für Reizstromtherapie vorbekannt, bei der auf einer Elektrodenplatte ein Metallrohr als Unterdruckdüse aufgelötet ist. Der Ansaugkanal ist dabei durch die Elektrodenplatte hindurch bis ins Innere des Rohrkanals nahe der engen Stelle gebohrt. Das die Unterdruckdüse bildende Metallrohr sowie die fest damit verbundene Elektrodenplatte sitzen in einem Elektrodengehäuse, das applikationsseitig eine umlaufende Dichtlippe aufweist. Wird die Saugelektrode mit der Dichtlippe auf der Hautoberfläche eines zu behandelnden Patienten aufgesetzt und wird dann anschließend vom einen Ende der Unterdruckdüse durch eine Gaspumpe beispielsweise elektrisch isolierendes Preßgas durch die Unterdruckdüse geblasen, so entsteht an der engen Stelle ein Unterdruck, der sich über den Ansaugkanal zur Applikationsstelle fortpflanzt. Die Saugelektrode saugt sich daraufhin auf der Hautoberfläche an. Elektrodenkontaktflüssigkeit, mit der z.B. zur Herstellung besseren Kontaktes ein zwischen Elektrodenplatte und Hautoberfläche des Patienten an der Applikationsstelle eingesetzter Elektrodenschwamm getränkt ist und die während der Behandlungszeit wegen des Unterdruckes über den Ansaugkanal in den Rohrkanal angesaugt wird, wird über das frei mündende Ende des Rohrkanals in die Atmosphäre versprüht. Die Elektrodenkontaktflüssigkeit kann also nicht in die Gaspumpe gelangen und diese verschmutzen.

Bei der bekannten Saugelektrode ist die elektrisch leitende Applikationselektrode mit der Unterdruckdüse fest verbunden. Außerdem sind Applikationselektrode und Unterdruckdüse im Elektroden gehäuse fest eingebaut, was zu Montage- und Fertigungsproblemen führt. Hierbei läßt sich der Ansaugkanal im Falle einer Verschmutzung nur schwierig reinigen, da er durch die Elektrode hindurch kaum zugänglich ist und nicht durchstoßen werden kann. Andererseits müssen in Fällen, bei denen unterschiedlich große, elektrisch leitende Elektroden eingesetzt werden sollen (z.B. in der Reizstromtherapie, unterschiedliche Therapie an unterschiedlichen Körperstellen), für jede Elektrodengröße komplette Sätze mit verschiedenen Saugelektroden bereitgestellt werden.

Aus der DE-C-33 00 765 ist eine Saugelektrode der eingangs genannten Art mit einem Gehäuse bekannt, in dem eine elektrisch leitende Applikationselektrode und eine Unterdruckdüse angeordnet sind, wobei die Unterdruckdüse einen Kanal mit einer verengten Stelle zur Erzeugung eines Unterdruckes aufweist. Die bekannte Vorrichtung ist als Strahlpumpe ausgebildet und mit der Elektrodeneinrichtung zu einer baulichen Einheit verbunden, wobei das Gehäuse gleichzeitig eine Saugglocke bildet. Dazu ist die Applikationselektrode fest an einem die Seitenwand der Saugglocke diametral durchstoßenden Strahlrohr aus Metall angeordnet und die Verengung des Strahlrohres steht über einen Ansaugkanal mit dem Inneren der Saugglocke in Verbindung. Da der Ansaugkanal nur durch eine Bohrung im Strahlrohrmantel in das Strahlrohr einmündet und der Ansaugkanal von der Applikationselektrode weitgehend überdeckt wird, ist die Reinigung des Ansaugkanals der bekannten Vorrichtung entsprechend schwierig.

Aufgabe vorliegender Erfindung ist es, eine Saugelektrode zu schaffen, die eine leichte Reinigung des Ansaugkanals ermöglicht und die Vielfalt erforderlicher Saugelektroden auf eine Mindestzahl begrenzbar macht.

Die Aufgabe wird erfindungsgemäß durch die Merkmale des Patentanspruchs 1 gelöst.

Gemäß der Erfindung ist das der Applikationselektrode zugewandte Ende des Ansaugkanals nach Abnahme der Elektrode leicht von außen zugänglich und kann durchstoßen werden, so daß der Ansaugkanal auch leichter als bisher zu reinigen ist. Aufgrund der abnehmbaren Applikationselektrode lassen sich jetzt in einem Elektrodengehäuse Elektroden mit unterschiedlichen Größen einsetzen. Damit ist eine Vielfalt von Applikationselektroden bei einer einzigen Saugelektrodenvorrichtung einsetzbar.

Das Elektrodengehäuse kann an der Applikationsstelle so dimensioniert sein, daß die größte im Gehäuse einzusetzende Applikationselektrode noch gut in das Saugelektrodengehäuse paßt.

In bevorzugter Ausgestaltung ist die Saugglocke an das Elektrodengehäuse ansteckbar und kann auch zusammen mit der jeweiligen Elektrode ausgewechselt werden. Damit ist die Saugglocke auch an die Größe der jeweils gerade eingesetzten Applikationselektrode anpaßbar.

Weitere Vorteile und Einzelheiten der Erfindung ergeben sich aus der nachfolgenden Beschreibung eines Ausführungsbeispiels anhand der Zeichnung und in Verbindung mit den Patentansprüchen 2 bis 7.

Es zeigen:
Figur 1 eine Saugelektrode, die eine erfindungsgemäße Unterdruckdüse umfaßt,
Figur 2 eine Saugglocke in Form einer auswechselbaren Haube für die Saugelektrode der Figur 1,
Figur 3 die Art der Befestigung der elektrisch leitenden Applikationselektrode an der Unterdruckdüse der Saugelektrode,
Figur 4 ein Ausführungsbeispiel der erfindungsgemäßen Unterdruckdüse im Längsschnitt,
Figur 5 eine Draufsicht auf die Unterdruckdüse der Figur 4, teilweise im Schnitt.

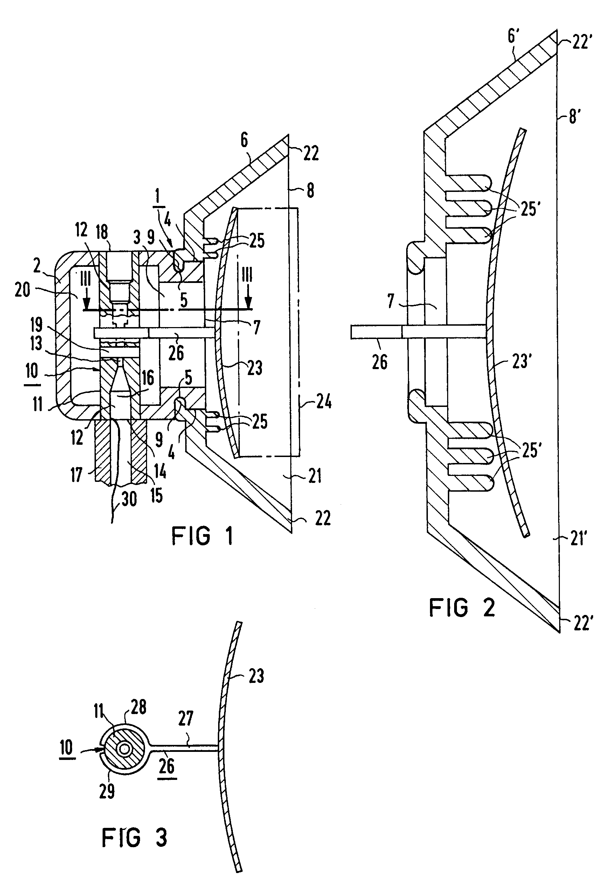

In der Figur 1 umfaßt die Saugelektrode 1 ein napfförmiges Gehäuse 2 (aus Kunststoff) mit einer proximalen Öffnung 3 und einem proximalen Ringwulst 4 mit einer Ringnut 5. Die Saugelektrode 1 umfaßt ferner eine als Haube ausgebildete Saugglocke 6 (aus elastischem Kunststoff, z.B. Silikongummi) mit einer distalen Öffnung 7 und einer größeren proximalen Öffnung 8. Die Saugglocke 6 ist mittels eines zum Ringwulst 4 des Gehäuses 2 passenden distalen Ringwulstes 9 in die Ringnut 5 des Ringwulstes 4 des Gehäuses 2 einknöpfbar. Die Figur 1 zeigt z.B. eine Saugelektrode 1, bei der am napfförmigen Gehäuse 2 eine Saugglocke mit relativ kleiner proximaler Öffnung 8 angeknöpft ist. Anstelle dieser Haube 6 (Saugglocke) kann auch eine beliebige andere Haube 6' mit z.B. sehr viel größerer proximaler Öffnung 8' angeknöpft werden. Eine solche Haube ist z.B. in der Figur 2 dargestellt.

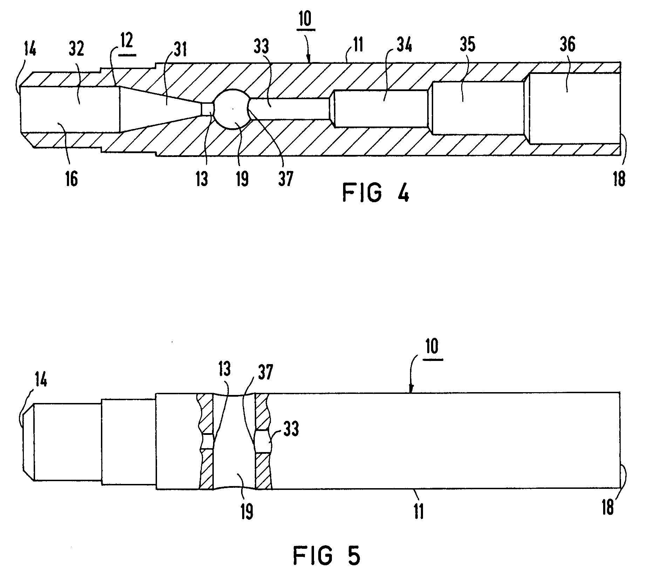

Die Saugelektrode der Figur 1 umfaßt eine Unterdruckdüse 10. Die Unterdrückdüse 10 besteht, wie in den Figuren 4 und 5 noch deutlicher dargestellt ist, aus einem (metallischen) Rohr 11, dessen Rohrkanal 12 an der Stelle 13 enger ist als an den anderen Stellen. Diese enge Stelle 13 dient zur Erzeugung eines Unterdrucks, wenn z.B. vom Rohrende 14 her über einen pneumatischen Anschluß 15 (elektrisch isolierendes) Preßgas durch den mit 16 bezeichneten Eingangsteil des Rohrkanals 12 durch die enge Stelle 13 gepreßt wird. Zum Anschließen einer geeigneten Gaspumpe dient ein pneumatisches Anschlußkabel 17. Der mit 18 bezeichnete Teil des Rohrkanals 12 mündet im Freien. Hier kann also das durch den Rohrkanal 12 gepreßte Gas in die Atmosphäre entweichen.

Gemäß der Figur 1 umfaßt die Unterdruckdüse 10 auch noch einen Ansaugkanal 19, der den Rohrkanal 12 in Höhe der engen Stelle 13 senkrecht durchstößt. Der Ansaugkanal 19 überträgt den an der engen Stelle 13 erzeugten Unterdruck durch den hohlen Innenraum 20 des napfförmigen Gehäuses 2 der Saugelektrode und dessen proximale Öffnung 3 in den hohlen Innenraum 21 der Haube 6. Dadurch saugt sich die Saugelektrode 1 nach Aufsetzen mit dem Rand 22 (bzw. 22') der Haube 6 (bzw. 6') auf die Hautoberfläche (nicht dargestellt) eines zu behandelnden Patienten an der Hautoberfläche fest.

Zum Übertragen eines Reizstromes auf die Hautoberfläche dient eine elektrisch leitende Elektrode 23 (bzw. 23') und ein zwischen Elektrode 23 (bzw. 23') und der Hautoberfläche an der Applikationsstelle einzubringender Schwamm oder Vlies 24, das mit einer geeigneten Elektrodenkontaktflüssigkeit (z.B. Leitungswasser oder Kochsalzlösung) getränkt ist. Die Elektrode 23 (bzw. 23') kann selbstverständlich auch zur Abnahme von physiologischen Signalen, z.B. EKG od.dgl., verwendet werden. Sie ist im vorliegenden Fall plattenförmig ausgebildet und leicht konvex gekrümmt.

Um zu verhindern, daß während des Ansaugvorganges die Elektrode 23 (bzw. 23') den Luftweg von der proximalen Öffnung 3 des napfförmigen Gehäuses 2 bis zur proximalen Öffnung 8 (bzw. 8') der Haube 6 (bzw. 6') verschließt, kann entweder die Elektrode selbst in diesem Bereich mit Öffnungen zur Weiterleitung des Unterdruckes versehen sein oder aber es kann z.B. (wie in der Figur 1 dargestellt) die Haube 6 (bzw. 6') mit Abstandshaltern 25 (bzw. 25') versehen sein, die die Elektrode 23 (bzw. 23') auf Distanz halten.

Die Elektrode 23 (bzw. 23') ist mittels einer elektrisch leitenden (metallischen) Anklemmfeder 26 an der metallischen Unterdruckdüse 10 in der in Figur 1 sowie auch in Figur 3 dargestellten Weise federnd und kontaktierend angeklemmt. Gemäß der Figur 3 umfaßt die Anklemmfeder 26 dazu ein Stielteil 27, das mit der Elektrode 23 verbunden ist und zwei mit dem Stielteil 27 verbundene, sich gabelnde Enden 28, 29, die als Federn ausgebildet sind (bei entsprechender Ausbildung der Anklemmfedern kann aber auch auf das Stielteil verzichtet werden). Diese Art der Befestigung der Elektrode hat den Vorteil, daß zum Reinigen des Ansaugkanals 19 (durch Stechen mit einer feinen Nadel) zuvor die Elektrode 23 (bzw. 23') aus dem Gehäuse 2 mit Haube 6 (6') entfernt werden kann, so daß man freien Zutritt zum Ansaugkanal 19 der Unterdruckdüse 10 hat. Darüber hinaus kann die jeweilige Elektrode jedoch jederzeit leicht durch eine andersartig gestaltete Elektrode, z.B. die Elektrode 23 in Figur 1 durch die in der Figur 2 dargestellte großflächigere Elektrode 23', ausgewechselt werden, wenn nämlich z.B. auch eine größere Haube 6' eingesetzt werden soll. Schließlich ist die beschriebene Art des Anklemmens einer Elektrode 23 bzw. 23' wegen der gelenkigen Verbindung auch noch vorteilhaft, da dadurch die Anpaßfähigkeit der Elektrode an z.B. abnorm gekrümmte Applikationsstellen verbessert wird. Die jeweilige Elektrode 23 bzw. 23' kann von der proximalen Öffnung 8 bzw. 8' der jeweiligen Haube 6 bzw. 6' durch die proximale Öffnung 3 des napfförmigen Gehäuses 2 hindurch an der Unterdruckdüse 10 angeklemmt werden.

Die Anklemmfeder 26 ist im vorliegenden Fall gleichzeitig Kontaktübertrager von der Elektrode 23 (bzw. 23') zum metallischen Rohr 11 der Unterdruckdüse 10. Von dort wird der Kontakt zur Signalleitung 30 weitergeleitet, über die entweder Reizstrom für die Elektrode 23 (bzw. 23') eingespeist wird oder elektrische Signale von der Elektrode abgenommen werden können.

Die Unterdruckdüse 10 selbst kann bei Bedarf im Inneren des napfförmigen Gehäuses 2 auswechselbar eingesetzt sein. Falls eine irreparabel defekte Unterdruckdüse 10 durch eine intakte ersetzt werden soll, so braucht nicht die Unterdruckdüse zusammen mit dem napfförmigen Gehäuse 2 weggeworfen zu werden. Vielmehr braucht lediglich im alten napfförmigen Gehäuse 2 die defekte durch eine intakte Unterdruckdüse ersetzt zu werden.

Beim Verschmutzen des Ansaugkanals 18 (z.B. dadurch, daß angesaugte Elektrodenkontaktflüssigkeit zusammen mit Staub den Ansaugkanal verstopfen) braucht, wie zuvor bereits erwähnt, der Ansaugkanal lediglich mit einer feinen Nadel durchstochen zu werden. Der sich dabei lösende Schmutz wird entweder durchgestoßen oder vor die Mündung der engen Stelle 13 des Rohrkanals 12 gedrückt und von dort vom durchgepumpten Gas durch den Teil 18 des Rohrkanals hindurch in Richtung des frei mündenden Auslaufs mitgerissen.

Die Figuren 4 und 5 zeigen die Unterdruckdüse 10 im vergrößerten Maßstab. Man sieht, daß das Eingangsteil 16 des Rohrkanals 12, das die enge Stelle 13 umfaßt, sich von der engen Stelle 13 in Richtung Rohrende 14 (wo das Preßgas eingedrückt wird) zuerst konusförmig (etwa unter dem Winkel 20°) erweitert (Konuskammer 31) und sich dann mit gleichbleibendem Durchmesser (Zylinderkammer 32) zum Ende 14 hin erstreckt. Der frei mündende Teil 18 des Rohrkanals 12 erweitert sich stufenförmig vom Ansaugstutzen 19 in den vier Zylinderkammern 33, 34, 35 und 36. Der Durchmesser der Zylinderkammer 33, der dem Durchmesser der Mündung 37 am Ansaugstutzen 19 entspricht, ist größer als der Durchmesser der engen Stelle 13 (z.B. etwa doppelt so groß).

## Patentansprüche

1. Saugelektrode (1) mit einem Elektrodengehäuse (2), einer elektrisch leitenden Applikationselektrode (23), einer Unterdruckdüse (10) und einer Saugglocke (6) aus elastischem Material, wobei die Unterdruckdüse einen Kanal (12) aufweist, der zumindest an einer Stelle (13) enger als an anderen Stellen zur Erzeugung eines Unterdrucks beim Durchführen eines Mediums ausgebildet und mit einem in Höhe der engen Stelle mündenden Ansaugkanal (19) versehen ist, derart, daß die Applikationselektrode (23) mittels einer elektrisch leitenden Anklemmfeder (26) in getrennter Anordnung von und lösbar an der Unterdruckdüse (10) befestigbar ist, daß die Saugglocke (6) lösbar an das die Unterdruckdüse aufnehmende Elektrodengehäuse (2) ansetzbar ist und daß bei abgenommener Applikationselektrode der Ansaugkanal (19) frei zugänglich ist.

2. Saugelektrode nach Anspruch 1, **gekennzeichnet durch** auswechselbare Applikationselektroden (23, 23') und/oder Saugglocken (6, 6') von unterschiedlicher Größe und/oder Form.

3. Saugelektrode nach Anspruch 1, **dadurch gekennzeichnet,** daß die Anklemmfeder (26) ein Stielteil (27) umfaßt, das mit dem einen Ende mit der Applikationselektrode (23, 23') verbunden ist und das am anderen Ende sich gabelnde Federstücke (28, 29) zum Anklemmen an die Unterdruckdüse (10) aufweist.

4. Saugelektrode nach Anspruch 1, **dadurch gekennzeichnet,** daß die Unterdruckdüse (10) in das napfförmig ausgebildete Elektrodengehäuse (2) auswechselbar eingesetzt ist.

5. Saugelektrode nach einem der Ansprüche 1 bis 4, **dadurch gekennzeichnet,** daß an der proximalen Öffnung (3) des napfförmigen Elektrodengehäuses (2) eine Ringwulst (4) vorgesehen ist und daß die Saugglocke (6) mit einem dazu passenden distalen Ringwulst (9) gegen eine Ringnut (5) des Elektrodengehäuses einknöpfbar ist.

6. Saugelektrode nach einem der Ansprüche 1 bis 4, **dadurch gekennzeichnet,** daß die Saugglocke (6, 6') Abstandshalter (25, 25') aufweist, die verhindern, daß die Applikationselektrode (23, 23') gegen die Unterdruckdüse (10) ansaugbar ist.

7. Saugelektrode nach einem der Ansprüche 1 bis 6, **dadurch gekennzeichnet,** daß an ein metallisches Rohr (11) der Unterdruckdüse (10) eine Signalleitung (30) zur Reizstromzuführung oder zur elektrischen Signalableitung angeschlossen ist, daß die Anklemmfeder (26) kontaktierend an das Rohr (11) angeklemmt ist und daß die Applikationgselektrode (23, 23') über das elektrisch leitende Stielteil (27) der Anklemmfeder mit dem Rohr (11) der Unterdruckdüse (10) sowie der Signalleitung (30) elektrisch leitende verbunden ist.

## Claims

1. Suction electrode (1) having an electrode housing (2), an electrically conductive application electrode (23), a vacuum nozzle (10) and a suction bell (6) of resilient material, wherein the vacuum nozzle has a duct (12) which is constructed to be narrower at least at one point (13) than at other points in order to produce a low pressure when conveying a medium, and is provided with a suction duct (19) opening on a level with the narrow point so that the application electrode (23) can be secured by means of an electrically conductive clamping spring (26) in a separate arrangement from, and detachably on, the vacuum nozzle (10), in that the suction bell (6) can be placed detachably onto the electrode housing (2) receiving the vacuum nozzle, and in that when the application electrode is removed the suction duct (19) is freely accessible.

2. Suction electrode according to claim 1, characterised by removable application electrodes (23, 23') and/or suction bells (6, 6') of various size and/or shape.

3. Suction electrode according to claim 1, characterised in that the clamping spring (26) includes a stem part (27) which is connected with one end to the application electrode (23, 23') and which has spring portions (28, 29) forking at the other end for clamping to the vacuum nozzle (10).

4. Suction electrode according to claim 1, characterised in that the vacuum nozzle (10) is removably inserted into the basin-shaped electrode housing (2).

5. Suction electrode according to one of claims 1 to 4, characterised in that on the proximal aperture (3) of the basin-shaped electrode housing (2) there is provided an annular enlargement (4), and in that the suction bell (6) having a distal annular enlargement (9) fitted to it can be pressed against an annular groove (5) of the electrode housing.

6. Suction electrode according to one of claims 1 to 4, characterised in that the suction bell (6, 6') has spacers (25, 25') which prevent the application electrode (23, 23') being sucked against the vacuum nozzle (10).

7. Suction electrode according to one of claims 1 to 6, characterised in that a signal line (30) is connected to a metallic pipe (11) of the vacuum nozzle (10) for carrying the stimulant current or for electrical signal conducting, in that the clamping spring (26) is clamped in contacting manner to the pipe (11) and in that the application electrode (23, 23') is connected by means of the electrically conductive stem portion (27) of the clamping spring to the pipe (11) of the vacuum nozzle (10) and to the signal line (30).

## Revendications

1. Électrode d'aspiration ou à succion (1) comportant un boîtier d'électrode (2), une électrode d'application électriquement conductrice (23), une buse à dépression (10) et une cloche aspirante (6) réalisée en un matériau élastique, la buse à dépression comportant un canal (12), qui, au moins en un emplacement (13), est plus étroit qu'en d'autres emplacements de manière à produire une dépression lors du passage d'un fluide, et comporte un canal d'aspiration (19) débouchant au niveau de l'emplacement étroit de telle sorte que l'électrode d'application (23) peut être fixée, au moyen d'un ressort d'accrochage électriquement conducteur (26) dans une disposition séparée de la buse à dépression (10) et de façon amovible de cette buse, que la cloche aspirante (6) peut être montée de façon amovible sur le boîtier d'électrode (2) qui loge la buse à dépression et que, lorsque l'électrode d'application est retirée, le canal d'aspiration (19) est librement accessible.

2. Électrode d'aspiration suivant la revendication 1, caractérisée par des électrodes d'application (23,23') et/ou des cloches aspirantes (6,6'), interchangeables, possédant des tailles et/ou des formes différentes.

3. Électrode d'aspiration suivant la revendication 1, caractérisée par le fait que le ressort de serrage (26) comporte une partie en forme de tige (27) qui est raccordée par une extrémité à l'électrode d'application (23,23') et qui possède, à son autre extrémité, des éléments de ressort (28,29), disposés en forme de fourche de manière à s'appliquer par serrage contre la buse à dépression (10).

4. Électrode d'aspiration suivant la revendication 1, caractérisée par le fait que la buse à dépression (10) est montée de façon interchangeable dans le boîtier d'électrode (2) réalisé en forme de bouton.

5. Électrode d'aspiration suivant l'une des revendications 1 à 4, caractérisée par le fait qu'un rebord annulaire (4) est prévu au niveau de l'ouverture proximale (3) du boîtier d'électrode en forme de bouton (2) et que la cloche aspirante (6) peut être fixée par encliquetage au moyen d'un rebord annulaire distal (9), qui lui est adapté, dans une gorge annulaire (5) du boîtier d'électrode.

6. Électrode d'aspiration suivant l'une des revendications 1 à 4, caractérisée par le fait que la cloche aspirante (6,6') comporte des entretoises (25,25'), qui empêchent que l'électrode d'application (23,23') puisse être appliquée par aspiration contre la buse à dépression (10).

7. Électrode d'aspiration suivant l'une des revendications 1 à 6, caractérisée par le fait qu'à un tube métallique (11) de la buse à dépression (10) est raccordé un conducteur de transmission de signaux (30) servant à appliquer un courant d'excitation ou à retransmettre électriquement un signal, que le ressort de serrage (26) est serré contre le tube (11) en étant en contact avec ce dernier et que l'électrode d'application (23,23') est raccordée d'une manière électriquement conductrice, par l'intermédiaire de la partie formant tige électriquement conductrice (27) du ressort d'accrochage, au tube (11) de la buse à dépression (10) ainsi qu'au conducteur de transmission de signaux (30).
